# EUROPEAN PATENT APPLICATION

(11) **EP 4 732 840 A1**
(43) Date of publication of application: **29.04.2026**
(21) Application number: 24825598.6
(22) Date of filing: 14.05.2024
(51) Int. Cl.: A61K 31/7032, A61K 8/60, A61P 31/04

(54) **AGENT FOR SUPPRESSING PROLIFERATION OF BACTERIA, AND COSMETIC, QUASI-DRUG, AND PHARMACEUTICAL COMPOSITION CONTAINING SAID AGENT, AND METHOD FOR SUPPRESSING PROLIFERATION OF BACTERIA**

(30) Priority: 20.06.2023 JP 2023100939
(71) Applicant: Toyo Sugar Refining Co., Ltd., Tokyo 1030006 (JP)
(72) Inventor: HANDA, Satoshi, Ichihara-shi, Chiba 290-0046 (JP); HASHIZUME, Yushi, Ichihara-shi, Chiba 290-0046 (JP); TANDIA, Mahamadou, Noda-shi, Chiba 278-0027 (JP)
(74) Representative: Dehns
(86) International application number: PCT/JP2024/017716
(87) International publication number: WO 2024/262189

(57) **Abstract**

The present disclosure relates to a proliferation inhibitor of at least one bacterium selected from the group consisting of the genus Gardnerella and the genus Atopobium, the inhibitor containing glyceryl glucoside.

## Description

### Technical Field

The present disclosure relates to a proliferation inhibitor for at least one bacterium selected from a group consisting of the genus Gardnerella and the genus Atopobium, a cosmetic, quasi-drug, and a pharmaceutical composition containing the same, and a method for inhibiting bacterial proliferation.

### Background Art

Various resident bacteria are present in the vagina of a healthy adult female, and the genus Lactobacillus is said to account for 75 to 95% thereof. The genus Lactobacillus (Döderlein bacillus), which is resident in the vagina, produces lactic acid, and prevents the invasion of extraneous bacteria by maintaining the vaginal pH at an acidic level of 4.5 or lower (Non Patent Literature 1).

As Döderlein bacilli, Lactobacillus acidophilus, Lactobacillus fermentum, Lactobacillus jensenii, Lactobacillus casei, Lactobacillus vaginalis, Lactobacillus crispatus, and Lactobacillus iners are known, and among these, Lactobacillus crispatus and Lactobacillus iners have been reported to be dominant bacteria in all four ethnic groups (Caucasian, Black, Hispanic, and Asian) (Non Patent Literature 2).

Vaginitis (vaginal disorder) is a disease concept characterized primarily by abnormal vaginal discharge, and representative examples thereof include bacterial vagionosis (BV), genital candidiasis, and trichomonal vaginitis. The causative microorganism of genital candidiasis is a fungus of the genus Candida, and the causative microorganism of trichomonal vaginitis is the protozoan Trichomonas vaginalis; however, bacterial vaginosis is caused by the disruption of the resident bacteria and is not caused by a specific causative microorganism.

In bacterial vaginosis, Döderlein bacilli decrease, and various aerobic and/or anaerobic bacteria abnormally proliferate. Although more than half of patients with bacterial vaginosis are asymptomatic, the main symptoms of bacterial vaginosis include malodorous vaginal discharge, gray to white discharge, burning sensation during urination, and itching around the vaginal area. Bacterial vaginosis is considered to increase the risk of pelvic inflammatory disease, miscarriage, preterm birth, and postpartum endometritis.

In bacterial vaginosis, known excessively proliferating aerobic bacteria include Streptococcus agalactiae, Escherichia coli, and Gardnella vaginalis, and known excessively proliferating anaerobic bacteria include Anaerocossus spp., the genus Atopobium such as Atopobium vaginae, Peptostreptococcus spp., Finegoldia spp., Micromonas spp., and Peptoniphilus spp.

Metronidazole is known as a drug having an effect of inhibiting the proliferation of bacteria mainly targeting Gardnella vaginalis, and is widely used for the treatment of bacterial vaginosis (Non Patent Literature 1).

However, bacteria belonging to the genus Atopobium are known to be resistant to metronidazole (Patent Literature 1), and the effect of metronidazole was not sufficient.

Meanwhile, glyceryl glycoside is a sugar derivative having a structure in which glucose is bonded to glycerin, and is a substance that has been conventionally used as a cosmetic raw material.

α-D-glucopyranosylglycerol, which is a type of glyceryl glucoside, has no skin irritation, has extremely excellent safety, and has been reported to have antimicrobial activity against Escherichia coli, Staphylococcus aureus, Pseudomonas aeruginosa, Pseudomonas cepacia, Staphylococcus epidermidis, and Corynebacterium minutissimum (Patent Literature 2).

However, in Patent Literature 2, the antimicrobial activity is evaluated based on the number of viable bacteria in a case where a sample collected after exposing bacteria to α-D-glucopyranosylglycerol for 24 hours or 48 hours after inoculation is cultured on a medium and the number of bacteria is counted, and how α-D-glucopyranosylglycerol acts on proliferating bacteria is not disclosed in Patent Literature 2. Further, how α-D-glucopyranosylglycerol acts on other bacteria, particularly bacteria of the genera Lactobacillus, Gardnerella, and Atopobium, is also not known.

### Citation List

### Patent Literature

Patent Literature 1: JP 2012-524117 W
Patent Literature 2: JP 2004-331577 A

### Non Patent Literature

Non Patent Literature 1: Bacterial Vaginosis and Sexually Transmitted Infections: Diagnosis and Treatment Guidelines, 2008, The Japanese Society for Sexually Transmitted Infections, p. 77-80, November 30, 2008
Non Patent Literature 2: "Vaginal microbiome of reproductive-age women", Jacques Ravel et al., Proc Natl Acad Sci USA (2011), 108 (Suppl 1); 4680-4687

### Summary of Invention

### Technical Problem

In view of the above background, the present inventors considered that, when it is possible to reduce the number of bacteria of the genus Gardnerella and the genus Atopobium without reducing the number of Döderlein bacilli, it would be preferable for maintaining the health of a site where at least one bacterium selected from the group consisting of bacteria of the genus Gardnerella and the genus Atopobium is present or suspected to be present, for example, the delicate zone, and for preventing or treating infections.

Therefore, an objective of the present disclosure is to provide a bacterial proliferation inhibitor that does not inhibit the proliferation of Döderlein bacilli and inhibits the proliferation of at least one bacterium selected from the group consisting of the genus Gardnerella and the genus Atopobium, a formulation containing the same, and a method for inhibiting bacterial proliferation.

### Solution to Problem

The present inventors have intensively studied to solve the above problems. As a result, the present inventors have found that the above problem can be solved by having the following configuration, and have completed the present invention.

The present invention relates to, for example, the following [1] to [7].
[1] A proliferation inhibitor of at least one bacterium selected from the group consisting of the genus Gardnerella and the genus Atopobium, the inhibitor containing glyceryl glucoside.
[2] The proliferation inhibitor according to [1], in which the bacterium is at least one selected from the group consisting of Gardnerella vaginalis and Atopobium vaginae.
[3] The proliferation inhibitor according to [1] or [2], which is used as a cleaning agent or a topical agent.
[4] A cosmetic or a quasi-drug comprising the proliferation inhibitor according to [1] or [2].
[5] A pharmaceutical composition for treatment or prevention of a disease involving Gardnerella vaginalis or Atopobium vaginae, the composition containing the proliferation inhibitor according to [2].
[6] The pharmaceutical composition according to [5], in which the disease is bacterial vaginosis.
[7] A method for inhibiting bacterial proliferation of at least one bacterium selected from the group consisting of the genus Gardnerella and the genus Atopobium, the method comprising a step of administering glyceryl glucoside to a subject.

### Advantageous Effects of Invention

According to the present disclosure, it is possible to provide a bacterial proliferation inhibitor that does not inhibit the proliferation of Döderlein bacilli and inhibits the proliferation of at least one bacterium selected from the group consisting of the genus Gardnerella and the genus Atopobium, a formulation containing the same, and a method for inhibiting bacterial proliferation.

Glyceryl glucoside has high safety since no toxicity is observed in any of the following tests: primary skin irritation test (rabbit), repeated skin irritation test, skin sensitization test (guinea pig intracutaneous Maximization method), phototoxicity test (guinea pig), photosensitization test, eye irritation test (rabbit), mutagenicity test (in vitro), and human patch test. Therefore, according to the present disclosure, it is possible to inhibit the proliferation of at least one bacterium selected from the group consisting of the genus Gardnerella and the genus Atopobium without concerns about safety or side effects.

### Brief Description of Drawings

Fig. 1 is a graph showing an actual measurement value (average value of n = 3) of turbidity in a bacterial proliferation test.
Fig. 2 is a graph showing a relative value (average value of n = 3) of turbidity in a bacterial proliferation test with respect to a control.

### Description of Embodiments

Next, the present invention will be specifically described.

The expression "A to B" in the numerical range means A or more and B or less unless otherwise specified. In addition, % means % by mass.

### <Glyceryl Glucoside>

In the present specification, glyceryl glucoside is also referred to as GG, and refers to a compound in which glycerin is bonded to one or more molecules of glucose via an α- or β-glycosidic bond.

Examples of the glyceryl glucoside include compounds represented by Formula (1) to Formula (5), and such compounds may be used alone or in combination of two or more thereof.

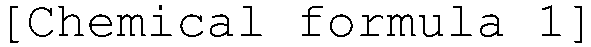

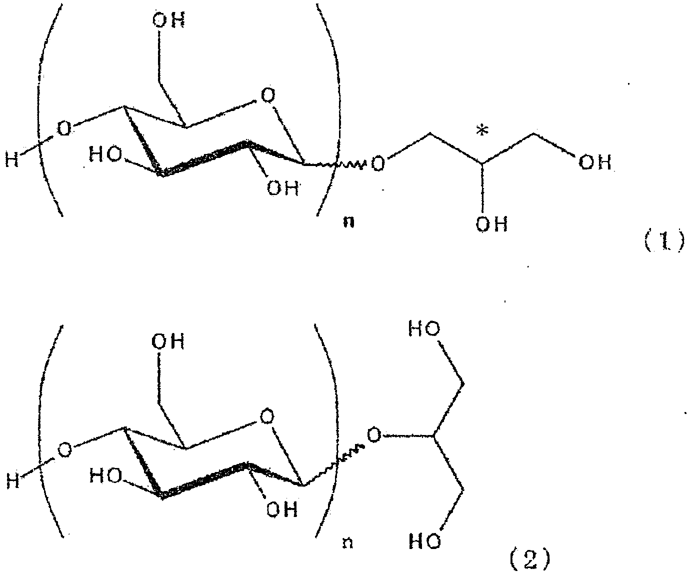

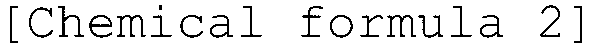

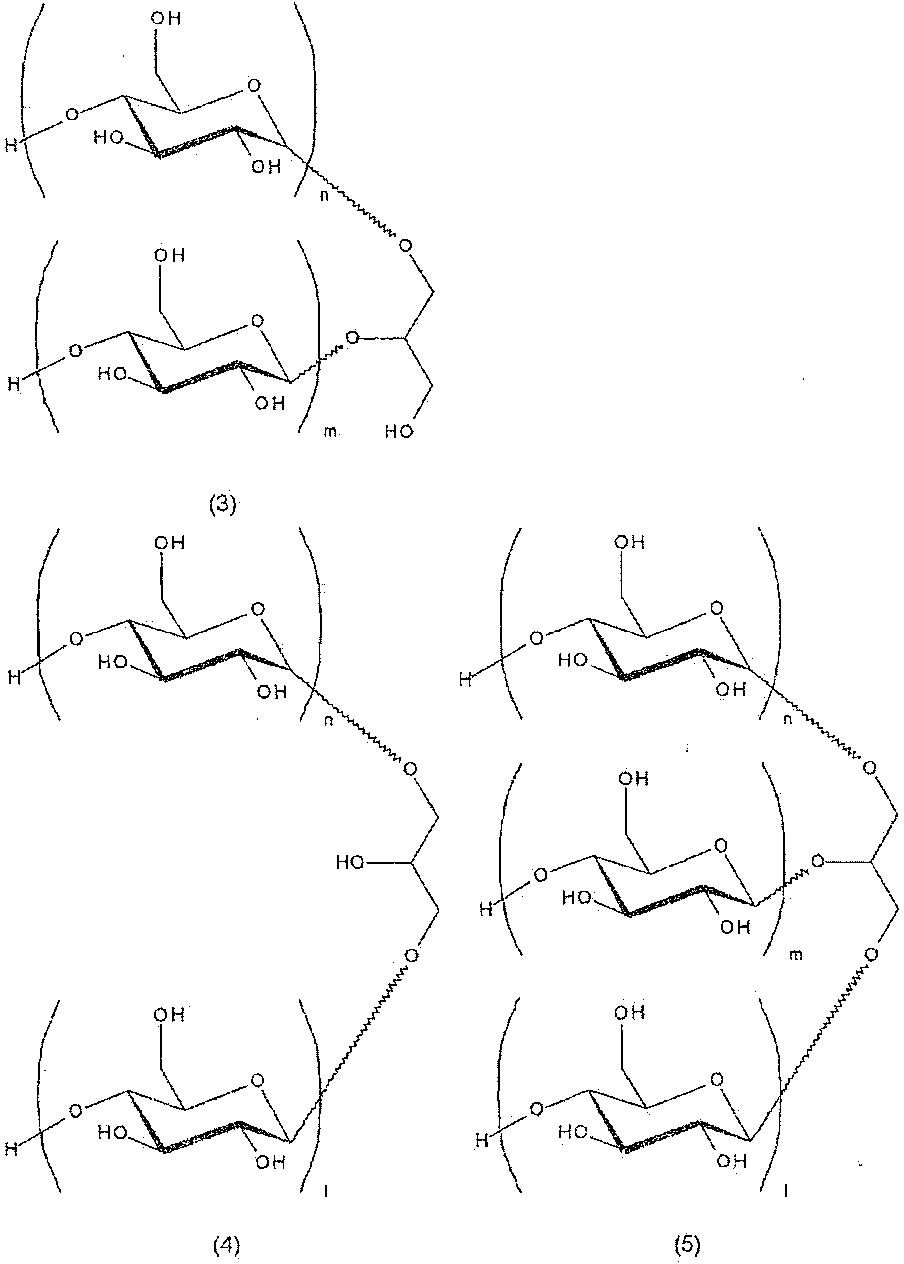

The compound represented by Formula (1) is 1-O-(α- or β-)-D-(mono or poly)glucopyranosylglycerol. The compound may also be referred to as 1-O-(α- or β-)-D-dihydroxypropyl(mono or poly)glucopyranoside.

The carbon atom at the 2-position marked with * in Formula (1) is a chiral carbon atom, and optical isomers, that is, the (2R)-form and the (2S)-form, exist.

In Formula (1), n is the degree of sugar condensation and represents an integer of 1 or more. In the commercially available glyceryl glucoside produced by a known method such as an enzymatic method or an organic synthesis method, n is usually an integer of 1 to 6, and most of the glyceryl glucoside is an integer of 1 to 3. In particular, a large number of compounds in which n is 1 are present in the GG composition.

The compound represented by Formula (2) is 2-O-(α- or β-)-D-(mono or poly)glucopyranosylglycerol. The compound may also be referred to as 2-O-(α- or β-)-D-dihydroxypropyl(mono or poly)glucopyranoside.

In Formula (2), n is the degree of sugar condensation and represents an integer of 1 or more. In the commercially available glyceryl glucoside produced by a known method such as an enzymatic method or an organic synthesis method, n is usually an integer of 1 to 6, and most of the glyceryl glucoside is an integer of 1 to 3. In particular, a large number of compounds in which n is 1 are present in the GG composition.

m and n in Formula (3), n and l in Formula (4), and l, m and n in Formula (5) are the degrees of sugar condensation and each independently represent an integer of 1 or more, and usually an integer of 1 to 6, most of which is an integer of 1 to 3, and particularly, compounds in which n, m, and l are 1 are abundantly present in the GG composition. In Formulae (3), (4), and (5), when n, m, and l are 2 or more, the glucopyranosyl groups may be linked by, for example, an α1-4 bond, a β1-4 bond, an α1-6 bond, or a β1-6 bond.

The glyceryl glucoside in the present invention preferably contains at least one of 1-O-D-glucopyranosylglycerol and 2-O-D-glucopyranosylglycerol represented by Formula (1) or Formula (2) in which n is 1. More specifically, the glyceryl glucoside preferably contains at least one selected from 1-O-α-D-glucopyranosylglycerol, 1-O-β-D-glucopyranosylglycerol, 2-O-α-D-glucopyranosylglycerol, and 2-O-β-D-glucopyranosylglycerol.

The glyceryl glucoside in the present invention may be one compound selected from 1-O-α-D-glucopyranosylglycerol, 1-O-β-D-glucopyranosylglycerol, 2-O-α-D-glucopyranosylglycerol, and 2-O-β-D-glucopyranosylglycerol, or may contain two or more compounds.

In addition, glyceryl glucosides of 1-O-α-D-glucopyranosylglycerol, 1-O-β-D-glucopyranosylglycerol, 2-O-α-D-glucopyranosylglycerol, and 2-O-β-D-glucopyranosylglycerol are those in which glycerin is bonded to one molecule of glucose, but in the present invention, glyceryl glucosides may be those in which glucose is further bonded, that is, those in which n is 2 or more, and may be a mixture of a compound in which n is 1 and a compound in which n is 2 or more.

For example, in the Formulae (1) to (5), when n, m, and l are each independently 2 or more, the bonding mode between the glucopyranosyl groups is not particularly limited.

In addition, the wavy lines in the Formulae (1) to (5) each independently represent that the hydroxyl group of glycerin may react with and bond to the hydroxyl group at the 1-position of the α-D-glucopyranosyl group (α bond), or may react with and bond to the hydroxyl group at 1-position of the β-D-glucopyranosyl group (β bond).

The glyceryl glucoside in the present invention preferably has a purity (mass ratio of components in which: in Formula (1) and Formula (2), n = 1 to 3; in Formula (3), n + m = 2 or 3; in Formula (4), n + l = 2 or 3; and in Formula (5), n = m = l = 1) of 70 to 100% by mass based on 100% by mass of the glyceryl glucoside. More preferably, the component in which n = 1 in Formula (1) and Formula (2) is 60 to 95% by mass, and even more preferably, 75 to 90% by mass. In addition, preferably, the glyceryl glucoside has 1-O-α-D-glucopyranosylglycerol as the main component, and more preferably, the 1-O-α-D-glucopyranosylglycerol is 10 to 50% by mass based on 100% by mass of the glyceryl glucoside.

Glyceryl glucoside may be used as a composition (also referred to as a GG composition) further containing, in addition to the compounds represented by the Formulae (1) to (5), a compound other than the compounds represented by the Formulae (1) to (5), for example, water, unreacted substances or reaction by-products (impurities) that may be mixed when preparing the compounds represented by the Formulae (1) to (5) by an organic synthesis method.

Examples of the impurities include glycerin blended as a raw material for producing glyceryl glucoside and a reaction by-product derived therefrom, and a glucose source and a reaction by-product derived therefrom. Examples thereof include alcohols such as ethanol, isopropyl alcohol, ethylene glycol, diethylene glycol, triethylene glycol, propylene glycol, dipropylene glycol, 1,3-butylene glycol, 1,2-pentanediol, glycerin, diglycerin, and polyglycerin, and sugars such as glucose.

The blending amount of glycerin in, for example, food and drink, seasoning, and cosmetics may be limited. Therefore, when the GG composition is used for the production of those products, the ratio of glycerin remaining in the GG composition is preferably 70% by mass or less, more preferably 50% by mass or less, still more preferably 45% by mass or less, particularly preferably 10% by mass or less, and most preferably 5% by mass or less, with respect to the total solid content (component excluding water) in the GG composition. Glycerin is preferably not contained, and may be 0% by mass, and is usually 10% by mass or less with respect to the total solid content in the GG composition.

The method for producing glyceryl glucoside in the present invention is not particularly limited, and glyceryl glucoside can be produced by a known method such as an enzyme method or an organic synthesis method. In addition, a commercially available product containing glyceryl glucoside can be purchased and used, and for example, "COSARTE-2G (registered trademark) (manufactured by Toyo Sugar Refining Co., Ltd.)", and "C-mo 2G (manufactured by Toyo Sugar Refining Co., Ltd.)" can be used.

In "COSARTE-2G", the content of glyceryl glucoside is about 66 to 78% by mass, and the content of glycerin is about 10% by mass or less in 100% by mass of "COSARTE-2G".

In "C-mo 2G", the content of glyceryl glucoside is about 30 to 38% by mass, and the content of glycerin is about 36 to 44% by mass in 100% by mass of "C-mo 2G".

Since glyceryl glucoside has good water solubility, glyceryl glucoside can be uniformly dissolved or dispersed even when glyceryl glucoside is added to water or a formulation containing a large amount of water. In addition, since glyceryl glucoside has a high moisturizing effect and low skin irritation, it can be suitably used for, for example, a delicate zone which is sensitive to irritation.

### <Proliferation Inhibitor>

According to an aspect of the present invention, the proliferation inhibitor contains glyceryl glucoside, and inhibits proliferation of at least one bacterium selected from the group consisting of the genus Gardnerella and the genus Atopobium.

The amount of glyceryl glucoside contained in the proliferation inhibitor is not particularly limited. For example, the lower limit of the amount of the glyceryl glucoside contained in the proliferation inhibitor is, for example, 20% by mass, 25% by mass, 30% by mass, 40% by mass, 45% by mass, 50% by mass, 60% by mass, 70% by mass, or 80% by mass. In addition, the upper limit of the amount of glyceryl glucoside contained in the proliferation inhibitor is, for example, 100% by mass, 99% by mass, 98% by mass, 95% by mass, 90% by mass, or 85% by mass. As the range of the amount of the glyceryl glucoside contained in the proliferation inhibitor, a range in which the lower limit and the upper limit are combined in any manner can be set in any manner, and for example, a range of 25 to 100% by mass, 30 to 90% by mass, or 60 to 80% by mass can be set. From the viewpoint of a proliferation inhibitory effect, the amount of the glyceryl glucoside contained in the proliferation inhibitor is preferably 20% by mass or more, and more preferably 30% by mass or more.

The proliferation inhibitor may contain glycerin, diglycerin, glucose, moisture, and other components as necessary.

Examples of the bacteria belonging to the genus Gardnerella include Gardnella vaginalis, and examples of bacteria of the genus Atopobium include, for example, Atopobium vaginae (Fannyhessea vaginae), Atopobium rimae, Atopobium parvulum, Atopobium fossor, Atopobium minutum, and Atopobium deltae. Since the bacterium is a main bacterium that excessively proliferate in bacterial vaginosis, the bacterium is preferably at least one selected from the group consisting of Gardnerella vaginalis and Atopobium vaginae among these bacteria.

The method for evaluating the effect of inhibiting proliferation of the bacteria is not particularly limited, and a known method can be used. For example, when the degree of proliferation of bacteria cultured by adding a test substance is lower than the degree of proliferation of bacteria cultured without adding a test substance under the same culture conditions, it can be evaluated that the test substance has an effect of inhibiting proliferation of bacteria.

More specifically, as described later in the Examples, when the number of bacteria cultured without adding the test substance is set to 100% and the number of bacteria cultured with the test substance added is less than 100%, it can be evaluated that the test substance has an effect of inhibiting proliferation of bacteria. In this case, the number of bacteria cultured by adding the test substance is preferably 90% or less, more preferably 80% or less, still more preferably 70% or less, and particularly preferably 60% or less with respect to the number of bacteria cultured without adding the test substance.

As the bacteria, preferably proliferating bacteria, and more preferably bacteria in the logarithmic growth phase, are used.

The method for measuring the number of bacteria is not particularly limited, and a known method can be used. The number of bacteria may be measured, for example, by a direct detection method for counting the number of bacteria such as measurement using a bacterial cell counting plate, a method of culturing on an agar plate and counting colonies, or a method of culturing on an agar plate and observing the presence or absence and size of the growth inhibition zone formed, or may be measured by an indirect method that quantitatively detects various indicators derived from the presence of bacteria, such as turbidity (absorbance) measurement using a spectrophotometer, a method of measuring the weight of bacterial cells, or a method of measuring bacterial components or biochemical activity.

For convenience, the number of bacteria is preferably measured by an indirect method, and more preferably turbidity measurement using a spectrophotometer.

### <Use of Proliferation Inhibitor>

The use of the proliferation inhibitor of the present invention is not particularly limited, but since the proliferation inhibitor has a high action of inhibiting the proliferation of at least one bacterium selected from the group consisting of the genus Gardnerella and the genus Atopobium, the proliferation inhibitor can be administered to a single or a plurality of living bodies, for example, for the purpose of maintaining health such as a delicate zone and/or for the purpose of preventing or treating infections.

The proliferation inhibitor is useful for the prevention or improvement of diseases or symptoms involving at least one bacterium selected from the group consisting of the genus Gardnerella and the genus Atopobium, and contributes to the prevention or improvement of the onset or progression of, for example, bacterial vaginosis, urinary tract infection, endometritis, acute salpingitis, postoperative infection, amniotic infection, infertility, preterm birth, and neonatal sepsis. The disease or condition is preferably bacterial vaginosis.

The dosage of the proliferation inhibitor may be appropriately selected depending on factors such as the type or severity of the disease or symptom involving at least one bacterium selected from the group consisting of the genus Gardnerella and the genus Atopobium, and the route of administration.

The administration period of the proliferation inhibitor may be appropriately selected depending on factors such as the type or severity of the disease or symptom involving at least one bacterium selected from the group consisting of the genus Gardnerella and the genus Atopobium, and the route of administration. It is preferably 3 to 80 days, and more preferably 7 to 60 days from the viewpoint of the proliferation inhibitory effect.

The route of administration of the proliferation inhibitor is not particularly limited, and a known administration route can be used, and oral or non-oral administration can be performed. Since administration is easy, the proliferation inhibitor is preferably administered non-orally, and more preferably applied topically.

When orally administered, the proliferation inhibitor may be used as a food or beverage in addition to a pharmaceutical product.

The proliferation inhibitor is preferably used as a cleaning agent or a topical agent from the viewpoint of the effect of inhibiting the proliferation of bacteria.

The target site of the cleaning agent or the topical agent is a site where at least one bacterium selected from the group consisting of the genus Gardnerella and the genus Atopobium is present or suspected to be present, and is preferably a female or male delicate zone, more preferably a female delicate zone, and still more preferably a vagina. In the present specification, the female delicate zone means the skin of the vulva, the vagina, and the periphery thereof, and the male delicate zone means the skin of the penis, the testicles, and the periphery thereof.

The topical agent means a formulation to be directly applied to a surface of, for example, skin or mucous membranes, and may be any of a pharmaceutical composition, a quasi-drug, and a cosmetic. The topical agent is not limited in terms of properties or dosage form, as long as it can be applied to or penetrate, for example, the skin or mucous membranes, and may be in the form of, for example, a solid, semi-solid, liquid, emulsion, cream, gel, mousse, spray formulation, or a two-component type in which these are used in combination.

The cleaning agent means a formulation for cleaning, for example, the skin and mucous membranes, and may be any of a pharmaceutical composition, a quasi-drug, and a cosmetic. The cleaning agent is not limited in terms of properties or dosage form, and may be in the form of, for example, a solid, semi-solid, liquid, emulsion, cream, gel, mousse, spray formulation, or a two-component type in which these are used in combination.

The cleaning agent may be a cleaning agent that contains a surfactant, and is typically used by foaming (for example, soap, body soap), or may be a cleaning agent that is filled in a cleaning device and used by spraying onto the cleaning target site.

The cleaning agent is preferably a vaginal cleaning agent. For example, the vaginal cleaning agent is filled in a container (vaginal cleanser) provided with a nozzle that can be inserted into the vagina and sprayed into the vagina to wash away residual blood and/or secretions remaining in the vagina or outside the vagina.

### <Formulation Containing Proliferation Inhibitor>

The proliferation inhibitor may be administered to a living body as it is, or may be administered as a formulation in which an effective amount of the proliferation inhibitor is blended together with a pharmaceutically acceptable carrier.

The dosage form of the formulation is not particularly limited, and may be any of a pharmaceutical product, a quasi-drug, and a cosmetic. In other words, one aspect of the present invention is a cosmetic or a quasi-drug containing the proliferation inhibitor. A further aspect of the present invention is a pharmaceutical composition containing the proliferation inhibitor.

The content of glyceryl glucoside in the formulation can be appropriately set according to the form and/or use of the formulation, and is, for example, 0.002% by mass or more, 0.003% by mass or more, 0.004% by mass or more, 0.005% by mass or more, or 0.006% by mass or more, and 30% by mass or less, 29% by mass or less, 28% by mass or less, 27% by mass or less, 26% by mass or less, 25% by mass or less, 24% by mass or less, 23% by mass or less, 22% by mass or less, 21% by mass or less, or 20% by mass or less from the viewpoint of the effect of inhibiting the proliferation of bacteria.

The cosmetic or quasi-drug is preferably a cosmetic or quasi-drug for a delicate zone, and more preferably a cream, ointment, gel, or lotion for a delicate zone.

In the cosmetics or quasi-drugs, components usually used in, for example, cosmetics, topical agents for skin which are pharmaceuticals, or cleaning agent, such as fats and oils, waxes, hydrocarbon oils, ester oils, higher alcohols, silicone oils, humectants, surfactants, watersoluble polymers, thickeners, powders, skin protecting agents, skin lightening agents, wrinkle improving agents, antiaging agents, plant extracts, preservatives, antiinflammatory agents, pH adjusting agents, sequestering agents, antioxidants, stabilizers, fragrances, dyes, and pigments can be appropriately blended as necessary as long as the effects of the present invention are not impaired. These components may be used alone or in combination of two or more thereof.

The cosmetic or quasi-drug may contain a probiotic such as Döderlein bacilli.

The pharmaceutical composition is preferably for treatment or prevention of a disease involving Gardnerella vaginalis or Atopobium vaginae.

Examples of the disease include bacterial vaginosis, urinary tract infection, endometritis, acute salpingitis, post-operative infection, amniotic fluid infection, infertility, premature birth, and neonatal sepsis, and bacterial vaginosis is preferable.

The dosage form of the pharmaceutical composition is not particularly limited, and may be a solid formulation such as a powder, granule, tablet, or capsule; or a liquid formulation such as a solution, suspension, or emulsion.

The pharmaceutical composition is preferably a pharmaceutical composition for a delicate zone, more preferably a vaginal suppository, cream, ointment, gel or lotion applied to skin or mucosa of a delicate zone, and still more preferably a vaginal suppository, cream, ointment, gel or lotion applied to skin or mucosa of a woman's delicate zone.

The vaginal suppository is a topical agent applied in the vagina, and may be of any dosage form such as a tablet, a capsule, an ointment, or a gel, or may be inserted into the vagina using an applicator.

The pharmaceutical composition may contain, for example, an antibiotic, an antifungal agent, a disinfectant, a local anesthetic, an antihistamine, an antipruritic agent, a preservative, a pH adjusting agent, an excipient, a binder, a disintegrant, a hydrating agent, a stabilizer, a solubilizing agent, a suspending agent, an isotonizing agent, a buffering agent, an antioxidant, and a probiotic such as Döderlein bacilli. These components may be used alone or in combination of two or more thereof.

Examples of the antibiotic include metronidazole, clindamycin, secnidazole, chloramphenicol, and nifuratel.

Examples of the antifungal agent include fluconazole, butoconazole, clotrimazole, miconazole, and tioconazole.

Examples of the disinfectant include benzalkonium chloride, benzethonium chloride, povidone-iodine, and isopropyl methylphenol.

Examples of the local anesthetic include lidocaine, xylocaine, dibucaine, and ethyl aminobenzoate.

Examples of the antihistamines include diphenhydramine, cetirizine, doxepin, and hydroxyzine.

Examples of the antipruritic agent include crotamiton.

The formulation can be produced by adding the proliferation inhibitor according to a method generally used for such a formulation. The proliferation inhibitor may be added at the initial stage of the production step of the formulation, or may be added at the middle or final stage of the production step, and the addition method may be appropriately selected from, for example, mixing, kneading, dissolution, immersion, dispersion, spraying, and application depending on the aspect of the formulation.

### <Method for Inhibiting Bacterial Proliferation>

One aspect of the present invention is a method for inhibiting bacterial proliferation of at least one bacterium selected from the group consisting of the genus Gardnerella and the genus Atopobium, the method comprising a step of administering glyceryl glucoside to a subject.

The method for administering glyceryl glucoside to a subject is not particularly limited, and any suitable means can be used depending on the subject. As the administration method, non-oral administration is preferable, and topical application to the skin or mucosa is more preferable. The topical application to the skin or mucous membranes may be, for example, a simple application method, a layered therapy, a sealing therapy, or a wetting therapy.

The target site to which glyceryl glucoside is administered is a site where at least one bacterium selected from the group consisting of the genus Gardnerella and the genus Atopobium is present or suspected to be present, and is preferably a female or male delicate zone, more preferably a female delicate zone, and still more preferably a vagina.

One aspect of the present invention is a therapeutic or preventive agent for a disease involving Gardnerella vaginalis or Atopobium vaginae, the agent containing glyceryl glucoside.

One aspect of the present invention is glyceryl glucoside for the use as a therapeutic or preventive agent for a disease involving Gardnerella vaginalis or Atopobium vaginae.

One aspect of the present invention is the use of glyceryl glucoside for inhibiting the proliferation of at least one bacterium selected from the group consisting of the genera Gardnerella and the genera Atopobium.

One aspect of the present invention is the use of glyceryl glucoside in the production of a proliferation inhibitor of at least one bacterium selected from the group consisting of the genera Gardnerella and the genera Atopobium.

One aspect of the present invention is a method for treating or preventing a disease involving Gardnerella vaginalis or Atopobium vaginae, the method comprising administering to the subject an effective amount of glyceryl glucoside.

### Examples

Next, the present invention will be described in more detail with reference to Examples, but the present invention is not limited thereto.

### <Example 1: Proliferation Test Using Gardnerella vaginalis, Atopobium vaginae, and Döderlein Bacilli>

### (Test Substance)

C-mo2G (manufactured by Toyo Sugar Refining Co., Ltd.) was used as glyceryl glucoside. Furthermore, as a positive control, penicillin-streptomycin (manufactured by FUJIFILM Wako Pure Chemical Corporation, No. 168-23191) was used. Sterile water was used as a control.

### (Cell Body and Medium)

Bacteria and media shown in Table 1 were used, and cultured under the conditions in Table 1.

**[Table 1]**

| Bacteria | ATCC No. | Medium | Culture condition | Number of culture days |
|---|---|---|---|---|
| *Lactobacillus crispatus* | 33820 | GAM semi-fluid high layer medium | 37°C, 5%CO₂ | 1 |
| *Lactobacillus iners* | 55195 | GAM semi-fluid high layer medium | 37°C, 5%CO₂ | 3 |
| *Gardnerella vaginalis* | 14018 | GAM semi-fluid high layer medium + 5% FBS | 37°C, 5%CO₂ | 3 |
| *Atopobium vaginae* | BAA-55 | GAM semi-fluid high layer medium + 5% FBS | 37°C, anaerobic | 2 |

As a GAM semi-fluid high layer medium, NO.05424 manufactured by NISSUI PHARMACEUTICAL CO., LTD. was used.

The number of culture days was set to such a number of days that bacterial proliferation could be visually confirmed in the control.

Various types of Döderlein bacilli are known, but since they are dominant bacteria in the vagina across all four ethnic groups (Caucasian, Black, Hispanic, and Asian), Lactobacillus crispatus and Lactobacillus iners were used as representative Döderlein bacilli in the experiment.

An experiment was performed using Atopobium vaginae as a representative bacterium of the genus Atopobium.

### (Pre-Culture)

Each bacterium in which a frozen stock (15% (vol/vol) glycerin was added to the medium) was inoculated into 9 mL of the medium, and culture was started. After culturing until reaching the logarithmic growth phase, the medium was well stirred to make the turbidity uniform, and then the absorbance at 600 nm was measured. The medium was diluted with a medium such that the absorbance (600 nm) was 0.1, and this was used as a bacterial diluent.

### (Test Method)

C-mo 2G was added to the medium at final concentrations of 0.5%, 1.0%, and 2.0%. Penicillin-streptomycin as a positive control was added to have a final concentration of 10% for Lactobacillus crispatus, a final concentration of 1% for Lactobacillus iners, a final concentration of 1% for Gardnerella vaginalis, and a final concentration of 10% for Atopobium vaginae.

200 µL of the bacterial diluent was added to 9 mL of the medium containing the test substance, and culture was started. After culturing, the medium was well stirred to make the turbidity uniform, and then the absorbance (turbidity) at 600 nm was measured. The test was performed at each point n = 3. The significant difference test was performed by a Student t-test, and *: p < 0.05, **: p < 0.01 relative to the control.

### (Results)

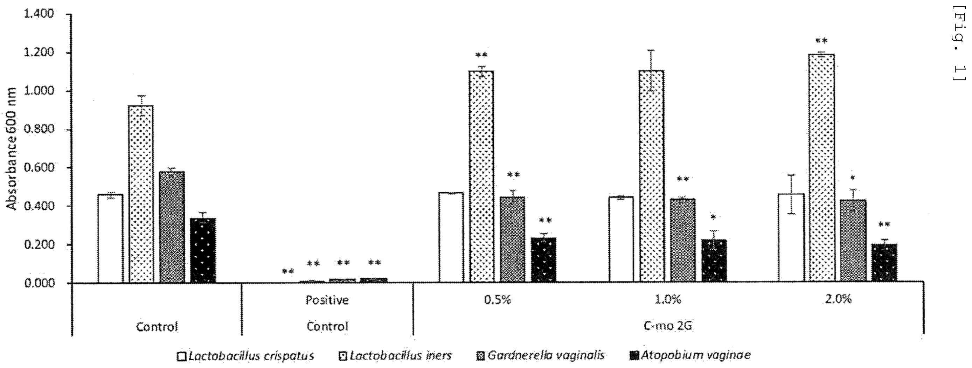

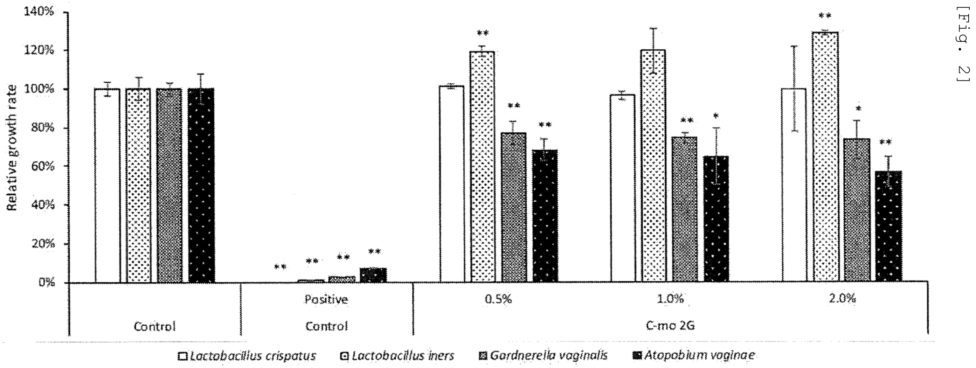

The results of the proliferation tests for each bacterium are shown in Figs. 1 and 2.

In Fig. 1, the vertical axis represents the actual measurement value (average value of n = 3) of absorbance (600 nm) (absorbance 600 nm). In Fig. 2, the vertical axis represents the relative growth rate, taking the average value absorbance (600 nm) of the control as 100%, and indicates the relative values of bacterial proliferation compared to the control.

In the positive control, the proliferation of all bacteria Lactobacillus crispatus, Lactobacillus iners, Gardnerella vaginalis, and Atopobium vaginae was strongly inhibited.

Addition of glyceryl glucoside resulted in little change in the number of Lactobacillus crispatus, but increased Lactobacillus iners relative to controls. Furthermore, when glyceryl glucoside was added, the proliferation of Gardnerella vaginalis and Atopobium vaginae was significantly inhibited, and this proliferation inhibitory effect showed a concentration dependence.

That is, it was revealed that glyceryl glucoside does not inhibit the proliferation of Lactobacillus crispatus and Lactobacillus iners, which are Döderlein bacilli, but inhibits the proliferation of Gardnerella vaginalis and Atopobium vaginae.

### <Reference Example 1: Proliferation Test Using Propionibacterium acnes, Streptococcus mutans, and Staphylococcus aureus>

### (Test Substance)

As glyceryl glucoside, COSARTE-2G (manufactured by Toyo Sugar Refining Co., Ltd.) was diluted with ion-exchanged water such that COSARTE-2G was 40% by mass. Further, as a positive control, BD (Sensi-Disc Tetracycline 30, Antimicrobial Susceptibility Test Kit, manufactured by Nippon Becton Dickinson Company, Ltd.) was used. As a control, glycerin (manufactured by Nacalai Tesque Inc.) was diluted with ion-exchanged water to be 40% by mass.

### (Propionibacterium Acnes)

Propionibacterium acnes (JCM 6425) was used as a representative strain of Propionibacterium acnes. A Propionibacterium acnes strain was added to GAM liquid medium and pre-cultured at 35°C under anaerobic conditions using an AnaeroPack.

### (Streptococcus Mutans)

Streptococcus mutans (JCM 5705) was used as a representative strain of Streptococcus mutans. A Streptococcus mutans strain was added to BHI liquid medium and pre-cultured at 35°C.

### (Staphylococcus Aureus)

Staphylococcus aureus (NBRC 13276) was used as a representative strain of Staphylococcus aureus. A Staphylococcus aureus strain was added to BHI liquid medium and pre-cultured at 37°C.

### (Test Method)

GAM agar plates and BHI agar plates were prepared. Pre-cultured Propionibacterium acnes, Streptococcus mutans, and Staphylococcus aureus strains in the logarithmic growth phase were suspended in liquid medium to a concentration of 1 × 10⁷ cells/mL, and 50 µL of this suspension was taken and spread onto each agar plate medium using a glass spreader.

Each test substance was sterilized by filtration using a membrane filter, and then 50 µL was impregnated into a sterilized paper disc. A paper disk was aseptically placed on an agar plate medium, and cultured for 48 hours under conditions suitable for each bacterial strain.

The effect of inhibiting the proliferation of bacteria was determined by the presence or absence and size of the growth inhibition zone around the paper disc.

### (Results)

In BD (positive control), a large growth inhibition zone was observed in all of Propionibacterium acnes, Streptococcus mutans, and Staphylococcus aureus, and the proliferation of these bacteria was strongly suppressed.

In glyceryl glucoside, no growth inhibition zone was observed in all of Propionibacterium acnes, Streptococcus mutans, and Staphylococcus aureus. That is, it was revealed that glyceryl glucoside did not inhibit the proliferation of Propionibacterium acnes, Streptococcus mutans, and Staphylococcus aureus. Glycerin used as a control also had the same result as glyceryl glucoside.

## Claims

1. A proliferation inhibitor of at least one bacterium selected from the group consisting of the genus Gardnerella and the genus Atopobium, the inhibitor containing glyceryl glucoside.

2. The proliferation inhibitor according to claim 1, wherein the bacterium is at least one selected from the group consisting of Gardnerella vaginalis and Atopobium vaginae.

3. The proliferation inhibitor according to claim 1 or 2, which is used as a cleaning agent or a topical agent.

4. A cosmetic or a quasi-drug comprising the proliferation inhibitor according to claim 1 or 2.

5. A pharmaceutical composition for treatment or prevention of a disease involving Gardnerella vaginalis or Atopobium vaginae, the composition containing the proliferation inhibitor according to claim 2.

6. The pharmaceutical composition according to claim 5, wherein the disease is bacterial vaginosis.

7. A method for inhibiting bacterial proliferation of at least one bacterium selected from the group consisting of the genus Gardnerella and the genus Atopobium, the method comprising a step of administering glyceryl glucoside to a subject.
